# EUROPEAN PATENT APPLICATION

(11) **EP 3 657 508 A1**
(43) Date of publication of application: **27.05.2020**
(21) Application number: 18462005.2
(22) Date of filing: 23.11.2018
(51) Int. Cl.: G16H 10/60, G06Q 10/10

(54) **SECURE RECRUITMENT SYSTEMS AND METHODS**

(71) Applicant: Healcloud Kft., 1061 Budapest (HU)
(72) Inventor: Farkas, Christopher Joseph, 1011 Budapest (HU)
(74) Representative: Danubia Patent & Law Office LLC

(57) **Abstract**

A computer-implemented method of identifying a person of interest in a computerized recruitment system is disclosed, wherein the recruitment system comprises a first server, a second server, a first database containing, for each person, a plurality of data records formed of application-specific person-related data and personally identifiable data associated with said person-related data, and a second database containing a plurality of de-identified data records, said second database being consistent with said first database. The method comprises the steps of specifying (200), at a first server, a query for searching persons of interest in the first database; sending (210) the query to a search engine residing at a second server; based on the query, obtaining (220), at the second server, at least one data record from the first database; at the second server, storing (230) the personally identifiable data record results in the second database; at the second server, de-identifying (240) the data records with generating a unique record identifier for each resulted data record; returning (250) the de-identified data records with an associated unique record identifier to the first server; selecting (260), at the first server, at least one data record from the de-identified data records according a to a predetermined selection scheme; sending (270) the record identifier of the selected at least one de-identified data record to the second server; at the second server, re-identifying (280) the selected at least one person of interest using the record identifier of the selected at least one de-identified data record and the set of personally identifiable data record results stored at the second server.

## Description

The present invention relates to secure recruitment systems and methods, in particular secure patient recruitment systems and methods for real world evidence (RWE) information collection, through schemes of extracting and managing personal data records.

Despite technological advancements, clinical trials still struggle to access sufficient eligible patients worldwide. Further, once potential candidates are located, patients are generally identified and selected through highly manual, resource, and labour-intensive processes, resulting in unnecessary expense, delays, errors, and even trial failure.

In late-phase, post-approval trials there is an increasing demand for RWE data to answer high-value questions regarding diagnosis, treatment, outcome, quality, and cost. For example, how efficient a particular drug or therapy is in everyday medical use. To date, there has been no automated technical solution able to collect such data at scale in Europe. Instead, RWE data has been largely gathered by reviewing patient files manually on site. Again, resulting in unnecessary expense, delays, errors, and even trial failure.

The document US 2005/0236474 A1 discloses a system for processing patient health information (PHI) while protecting the confidentiality of PHI to achieve regulatory compliance. The PHI contains patient medical data and associated patient identification data. A de-identification agent extracts patient medical data and creates de-identified patient information. A key is generated that allows subsequent re-association of the patient medical data and the patient identification data. The de-identified patient data base may be queried for patient screening purposes. Patient queries are processed only if the study or patient screening has been authorized by appropriate authorities (i.e., medical personnel). Patients whose medical characteristics conform with the patient query are selected for possible use in a study. If re-identification of the selected patients is necessary and authorized, the key may be used to provide the necessary re-association between the patient medical data and the respective patient identification data.

One of the drawbacks of the above system is that is does not provide a global data management for a particular patient who is treated in various health care sites, such as hospitals, clinics, physicians, etc. Due to the lack of a method to uniquely identify patients in the overall system, it is impossible to provide a global, holistic view of a patient on the basis of multiple patient medical records collected from various health care entities.

A further drawback of the above system is that the keys and the associated pieces of identification information are permanently stored together in a key file which may be thus exposed to a malicious attack.

It is an object of the present invention to eliminate at least one of the above mentioned and other deficiencies of the known data retrieval schemes that manage personal data records including personally identifiable data.

These and other objects are achieved by providing a first computer-implemented method of identifying a person of interest in a computerized recruitment system comprising a first server, a second server, a first database containing, for each person, a plurality of data records formed of application-specific person-related data and personally identifiable data associated with said person-related data, and a second database containing a plurality of de-identified data records, said second database being consistent with said first database, the method comprising:
a) specifying, at a first server, a query for searching persons of interest in the first database,
b) sending the query to a search engine residing at a second server,
c) based on the query, obtaining, at the second server, at least one data record from the first database,
d) at the second server, storing the personally identifiable data record results in the second database,
e) at the second server, de-identifying the data records while generating a unique record identifier for each resulted data record,
f) returning the de-identified data records with an associated unique record identifier to the first server,
g) selecting, at the first server, at least one data record from the de-identified data records according a to a predetermined selection scheme,
h) sending the record identifier of the selected at least one de-identified data record to the second server,
i) at the second server, re-identifying the selected at least one person of interest using the record identifier of the selected at least one de-identified data record and the set of identifiable data record results stored at the second server.

The above objects are further achieved by providing a second computer-implemented method of identifying a person of interest in a computerized recruitment system comprising a first server, a second server, a first database containing, for each person, a plurality of data records formed of application-specific person-related data and personally identifiable data associated with said person-related data, and a second database containing a plurality of de-identified data records, said second database being consistent with said first database, the method comprising:
a) specifying, at the second server, a query for searching persons of interest in the second database,
b) based on the query, obtaining de-identified data records from the second database,
c) at the first server, obtaining, from the first database, at least one personally identifiable data record matching the query, and
d) at the first server, re-identifying at least one person of interest based on the at least one personally identifiable data record.

Furthermore, the above objects are achieved by providing a first computerized recruitment system for identifying a person of interest, the system comprising:
a first server configured to perform the steps a), b), g) and h) of the first method;
a second server configured to perform the steps c) to f), and i) of the first method;
a first database coupled to said first server and containing, for each person, a plurality of data records formed of application-specific person-related data and personally identifiable data associated with said person-related data; and
a second database coupled to said second server and containing a plurality of de-identified data records, said second database being consistent with said first database.

The above objects are further achieved by providing a second computerized recruitment system for identifying a person of interest, the system comprising:
a first server configured to perform the steps c) and d) of the second method;
a second server configured to perform the steps a) and b) of the second method;
a first database coupled to said first server and containing, for each person, a plurality of data records formed of application-specific person-related data and personally identifiable data associated with said person-related data; and
a second database coupled to said second server (410) and containing a plurality of de-identified data records, said second database being consistent with said first database.

Providing access to data at scale through its system agnostic data extraction capabilities and de-identification algorithm, the present invention may be applied across a broad range of industries, e.g., in life sciences, insurance, financial services, social media and human resources.

Applied in the life sciences industry, the present invention enables life science researchers to access both retrospective and prospective medical data in a secure, auditable and legally compliant way, facilitating the selection of matching patients for early phase clinical trials and supporting RWE information collection for observational trials.

Furthermore, by assigning a unique anonymous identifier to each patient, the present invention allows researchers to see the entirety of patient pathways, i.e. the health status and treatment history of a given patient across various healthcare institutions.

The various embodiments of the systems and methods according to the present invention have at least one of the following novelties and advantages:
a) multiple local databases can store personally identifiable data records, such as medical records, for the same person with a standardized unique anonymous ID;
b) a central database can aggregate various personal records of the same person using the standardized unique anonymous ID associated with the given person;
c) the associations between the anonymous IDs and the personal IDs (personally identifiable information) are not permanently stored together;
d) the local re-identification of the persons is carried out by executing a central search query again in one or more local databases and the personally identifiable data of the matching data records are locally recovered and output to a person authorized by law (e.g. physician, therapeutist, etc).

The invention will now be described in detail with reference to the accompanying drawings in which:
Figure 1 is a schematic block diagram of a first embodiment of the system according to a first aspect of the present invention.
Figure 2 illustrates a flow diagram including the essential steps of the method according to the first aspect of the invention.
Figure 3 is a schematic block diagram of a second embodiment of the system according to the first aspect of the present invention.
Figure 4 illustrates a schematic block diagram of a third embodiment of the clinical trial patient recruitment system according to a second aspect of the present invention.
Figure 5 illustrates a flow diagram including the essential steps of the method of identifying a person of interest using the system shown in Figure 4.
Figure 6 illustrates a fourth embodiment of the system according to the second aspect of the invention.
Figure 7 is a flow diagram including the main steps of the method of identifying a person of interest using the system shown in Figure 6.
Figure 8 illustrates an example of the query descriptor code.

As will be discussed in greater detail herein, the computer systems and methods of operation described herein may be used to provide multiple levels of security and access to patient data. Access to patient data in a legally compliant manner is particularly important for patient identification and selection for the purpose of clinical trials and late phase observational studies. Although concern for patient privacy has always been an issue, the personal privacy in a broader sense has an increasing importance in many other fields like employment agencies, social networking services, etc, wherein sensitive personal data are stored and used for various purposes.

As used herein, the term "sensitive personal information" refers to any kind of personal information that is considered confidential and must therefore be protected. Sensitive personal information refers to any personally identifiable data, i.e. information that is directly or indirectly indicative to a person's identity.

The term "personally identifiable data record" should in particular be understood to mean data records that contain application-specific information and personally identifiable data about a person who is registered in a source database. For example, in the field of clinical recruitment, the personally identifiable data records are patient data records kept by one or more health institutions, in particular hospitals.

The term "de-identification routine" shall be understood as a routine intended to remove or mask, if appropriate, personally identifiable information from a registered person's data record. The de-identification routine may remove further data deemed appropriate by a person skilled in the art, which indirectly suggests the identity of the registered person and / or which is unnecessary for recruitment, from the personally identifiable data record. Furthermore, the de-identification routine may add one-to-one pseudonym information to the personally identifiable data records.

The term ""anonymizing" is understood to mean a process in which de-identified data records are generated and re-identification of the registered persons is not possible under reasonable circumstances.

The term "search result" refers to the information intended to convey to the user a result of his or her research. The search result preferably comprises at least the application-specific information and/or personally identifiable data. The search result preferably has at least information about a number of the personally identifiable data records found.

A "source database" is to be understood as meaning in particular a means of the recruitment system which is intended to permanently store the personally identifiable data records of the registered persons. The source database is typically designed as a database that appears appropriate to a person skilled in the art, for example as an SQL database or as an object-oriented database.

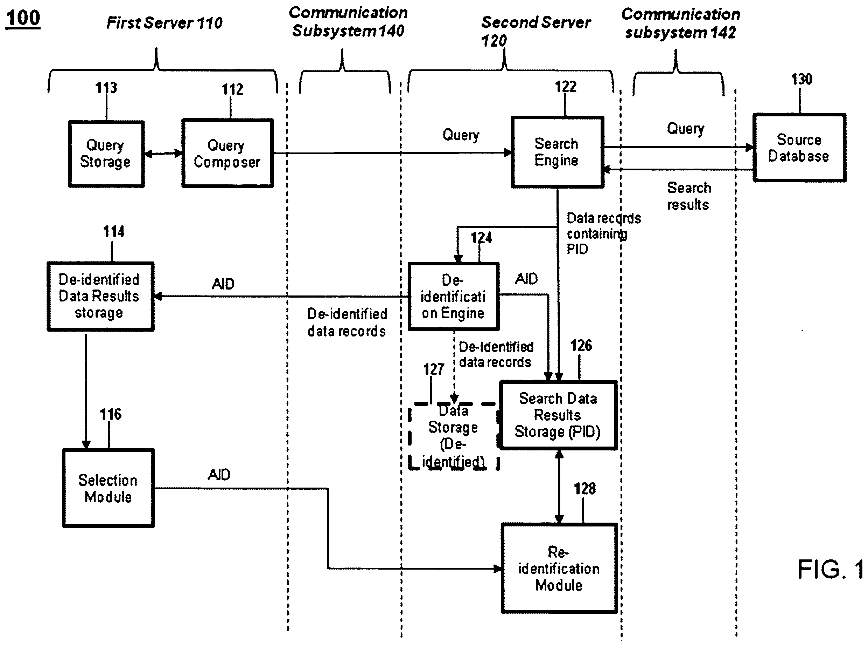

Figure 1 illustrates a schematic block diagram of the first embodiment of a recruitment system 100 according to a first aspect of the present invention. The recruitment system 100 comprises a first server 110, a second server 120 and a source database 130. The first server 110 and the second server 120 communicate to each other through a first communication subsystem 140, whereas the second server 120 and the source database 130 communicate to each other through a second communication subsystem 142. In some embodiments, the first and second communication subsystems 140, 142 are formed by a communication network, like the internet or an intranet. In this case, multiple first servers 110 may be connected to the second server 120 and multiple source databases 130 may be coupled to the second server 120 to form a highly distributed recruitment system. In other embodiments, only the first communication subsystem 140 is provided by a communication network, while the second communication subsystem 142 is a dedicated communication line between the second server 120 and the source database 130. This latter configuration is typical in the case where the source database 130 is arranged at the same site (e.g. hospital, employment agency, etc.) where the second server 120 is operating. Although for the sake of simplicity, Figure 1 depicts only one first server 110, one second server 120 and one source database 130, the recruitment system 100 may include any number of these entities. The actual configuration of the system 100 may depend on the specific field of application and is assumed obvious for those skilled in the art.

The first server 110 comprises a query composer 112 adapted for creating and managing search queries. A search query is preferably composed of a plurality of fields, each field relating to a searchable data element of the personally identifiable data records stored in the database 130. The search fields may specify a particular numeric value, a range of numeric values, a code of a personal feature, a string or a text element, etc.

The first server 110 further comprises a de-identified data storage 114 adapted for storing de-identified data records resulting from a search. The first server further comprises a selection module 116 allowing a user to select one or more de-identified data records from among the de-identified data records stored in the data storage 114 and to send an identifier of each selected data records to the second server 120 for re-identification.

The second server 120 comprises a search engine 122, a de-identification engine 124, a search result data storage 126 and a re-identification module 128. The search engine 122 is configured to receive a query from the first server 110 and to forward the query to a local source database 130 through a dedicated communication line or to a remote source database 130 via a communication network, e.g. internet, to find personally identifiable data records that match the query. The search engine 122 is further configured to receive the matching data records as search results containing personally identifiable data and to forward them to the search result data storage 126 and the de-identification engine 124. The de-identification engine 124 is adapted for running a de-identification routine to produce de-identified data records from the search results and to generate an anonymous ID for each search result. In some embodiments, the anonymous IDs are also permanently stored in the search result data storage 126 together with the associated identifiable data records.

The re-identification module 128 is adapted for receiving the one or more selected anonymous IDs from the first server 110 and to retrieve the corresponding data records from the search result data storage 124 for presenting the retrieved identifiable data records to an authorized person.

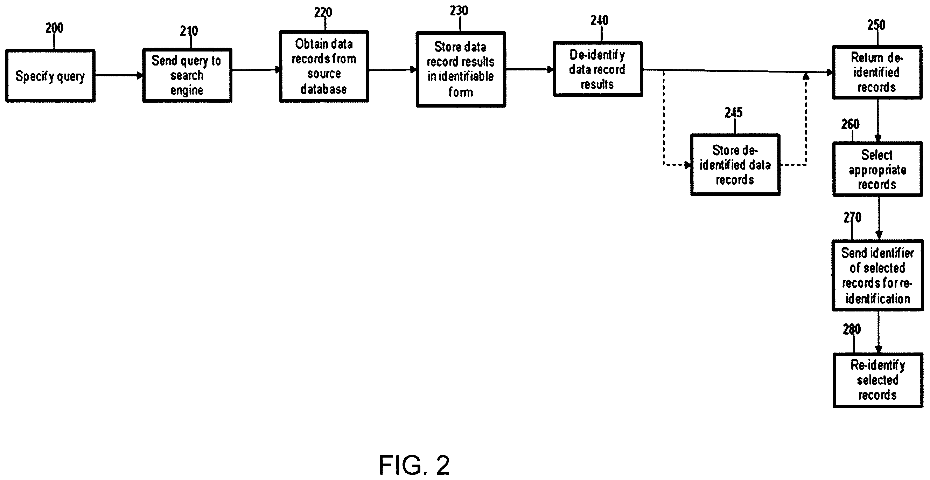

The method of identifying a person of interest using a database containing sensitive personal data (including personally identifiable data) according to the first aspect of the invention will now be described with reference to Figures 1 and 2, wherein Figure 2 illustrates a flow diagram including the essential steps of the method.

First, in step 200 a query is specified by the user, e.g., a researcher of a health institute, a recruitment administrator of a company, etc., at the first server 110 for searching persons of interest in the application-specific source database 130. For each person registered in the source database 130, application-specific person-related data and personally identifiable data associated with the application specific data are stored in the form of data records in the database 130.

Preferably, for each query that has been sent to the second server 120, a unique query ID is generated so that the queries can be managed at the first server 110 and tracked throughout the system 100. To this end, the full content of the executed queries and the associated query IDs are preferably stored at the first server 110 in a query storage 113.

As mention before, a query may be composed of multiple search fields specifying the actual values for various search parameters. Once the query has been completed, the query is forwarded to the search engine 122 of the second server 120 in step 210. The search engine 122 is configured to perform searches in any one or more of the source databases 130 coupled to the second server 120 and to receive the search result from the source database 130 currently used. Thus, in step 220, application-specific person-related data and associated personally identifiable information are obtained from the application-specific source database 130 based on the query. The returned data records relating to at least one person of interest together form a set of search results.

Next, in step 230, the currently received identifiable search results are stored in the search result data storage 126 of the second server 120. It means that any authorized operator of the second server 120 can access to and view the search results stored at the second server 120.

The search results (i.e. identifiable data records) are then also subject to de-identification in step 240, resulting in a set of de-identified data records in which the application-specific person-related data remains unchanged or is subject to masking so that the end users will be able to analyze the content of these data, while the personally identifiable data records is de-identified so that the end users be unable to directly identify or even guess the identity of the persons of interest who are associated with the de-identified data records.

In some embodiments of the method, for each de-identified data record, a record ID is generated, which uniquely identifies a de-identified data record stored in the search result data storage 126. Preferably, the record IDs and the corresponding query IDs are also stored in the search result data storage 126 together with the de-identified data records. A particular combination of a query ID and a record ID uniquely identify one data record in the overall system.

Alternatively or additionally, in some embodiments of the method, a unique anonymous ID may be generated for each person of interest whose data record has been returned as a search result. The anonymous ID is generated according to an algorithm that produces the same anonymous ID for a given person whose data records are stored in more than one source database 130. This scheme allows a global (or holistic) view of the patient pathways of individuals registered in various source databases 130. For example, when a patient has medical records at different hospitals where he or she has been treated with different diseases, his or her anonymous ID will always be the same for any de-identified data record obtained from different hospital databases. The use of this kind of unique anonymous ID for each person registered at any two or more sites in the system allows the aggregation of the different data records of the given person at the second server 120 and thus to form a global data record for that person, which highly improves efficiency and analysing capabilities of the clinical trial recruitment process.

Optionally, in step 245 the de-identified data records may also be stored together with the respective anonymous IDs in a de-identified data storage 127 at the second server 120 for also allowing certain operators of the second server 120 to access to and use the de-identified data records for statistical, business or other purposes.

As a next step 250, the de-identified data records as search results, along with the associated record identifiers, like the pairs of a query ID and a record ID, or an anonymous ID alone, are sent to the first server 110 for further processing by the end user.

In step 260, the end user of the first server 110 selects, by means of the selection module 116, at least one de-identified data record from among the de-identified data records received as a set of search results. The selection from the data records may be based on specific recruitment criteria applied by the end user of the first server 110. In case the query can be specified in a rather sophisticated way and the filtering criteria can be set to precisely define the required features of the persons of interest, it is expected that all of the search results relate to appropriate persons of interest and therefore the selection procedure results in the selection of all data records. In other embodiments, wherein the query cannot be specified precisely enough or the query is intentionally specified in a relatively broad manner, the end user may apply further selection criteria for the search results to select a subset of the de-identified data records for re-identification.

After the selection has been made in step 260, the end user sends, by means of the selection module 116, the identification data of the selected at least one de-identified data record from the first server 110 to the second server 120 in step 270. As mentioned above, the identification data of the selected de-identified data records may include the query ID and the record ID, or simply the anonymous ID belonging to the selected data records.

At the second server 120, the identifier of the selected de-identified data records are received by the re-identification module 128, which retrieves the respective search result data records (stored in an identifiable form) from the search result data storage 126 for presentation to an authorized operator of the second server 120 in step 280. Using the personally identifiable information of the data records identified on the basis of the record identifiers, the re-identification module 128 is configured to present personally identifiable information (e.g. name, contact information, consents, etc.) of the selected persons of interest. The authorized operator of the second server 120 can then directly contact the persons of interest to inform them about the final result of the recruitment process and the further steps of cooperation.

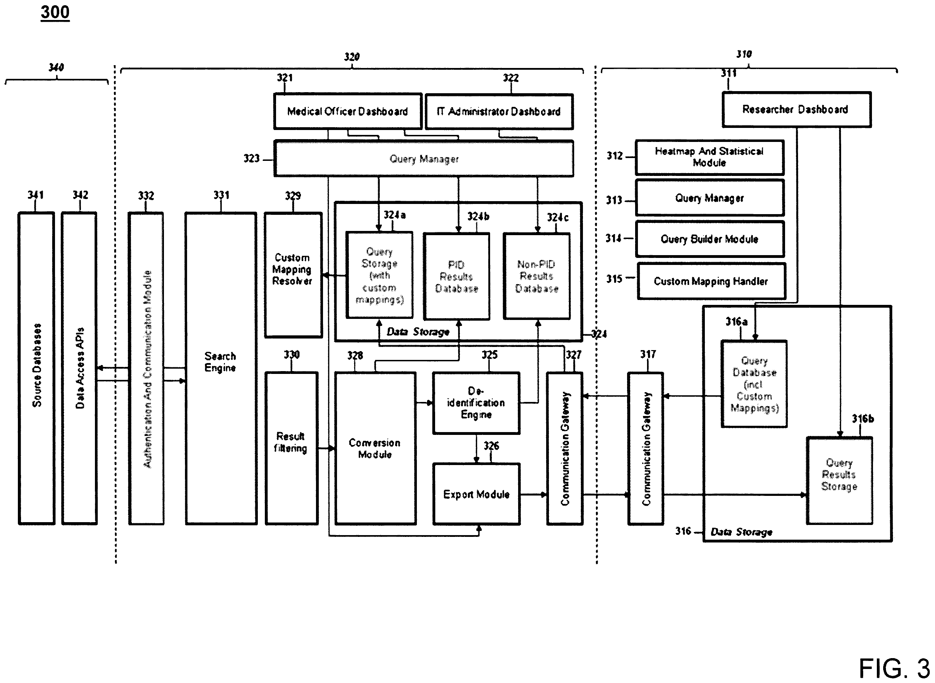

Figure 3 illustrates a second embodiment of the system according to the first aspect of the invention. This embodiment corresponds to a specific computer-implementation of the above described system in the medical field for a clinical trial recruitment scheme. As shown in Figure 2, the recruitment system 200 comprises additional modules and data storages to provide further functions with respect to the first embodiment of the system described with reference to Figure 1 for allowing an even more flexible and more secure operation of a clinical trial patient recruitment system according to the invention.

As shown in Figure 3, the recruitment system 300 includes at least one first server 310, at least one second server 320 and at least one database server 340, only one entity of them, respectively, is illustrated in Figure 3.

The first server 310 is operated by the end user, for example a researcher of a clinical research organization. The first server 310 may include a Researcher Dashboard 311, a Heatmap and Statistical Module 312, a Query Manager 313, a Query Builder Module 314, a Custom Mapping handler 315, a Data Storage 316 and a Communication Gateway 317.

The Researcher Dashboard 311 may be configured to allow a researcher to build and edit queries with the Query Builder Module 314, to manage query statuses and to request query execution with the Query Manager 313, to create special queries/requests using the Heatmap and Statistics Module 312 and to manage custom data mappings for the queries using the Custom Mapping Handler 315. The query requests/query versions are stored in the Data Storage 316, more particularly in its Query Database 316a.

Once the query request is formulated and marked as ready by the researcher using the Researcher Dashboard 311 (and reviewed by their supervisor, the Senior Researcher, if appropriate) it is forwarded to the second server 320 via the Communication Gateway 317. The Communication Gateway 317 preferably uses asymmetric encryption for the communication data flow.

At the second server 320, the incoming query requests (e.g., query packages) sent by the first server 310 are stored in the Data Storage 324, in particular in its Query Storage 324a. The query requests can be accessed to and processed by an authorized user of the second server 320, for example a Medical Officer and/or an IT Administrator by means of a Medical Officer Dashboard 321 and IT Administrator Dashboard 322, respectively. The Medical Officer may execute or reject the query request using the Medical Officer Dashboard 321.

Once the query is approved by the Medical Officer, the query is forwarded to the Custom Mapping Resolver 329 and then to the Search Engine 331. The Custom Mapping Resolver 329 may be configured to allow an authorized user of the second server 320 to use customized code lists to facilitate query building. The Search Engine 331 may be configured to divide the original query into fragments which can be processed by the database server 340. The Search Engine 331 generates a set of queries based on the query request sent by the researcher and determines which database records or what statistical data are to be returned to the researcher. The Search Engine 331 may also be configured to form the resulting data sets according to a predetermined format.

The queries are sent from the second server 320 through an Authentication and Communication Module 332 to the Database Server 340, in which the queries are received using a Data Access Application Programming Interface (API) 342 operatively coupled to the Source Database 341.

All resulting data sets read from the Source Database 341 are converted into a predetermined format by the Conversion Module 328 and then stored in the Data Storage 324, more particularly in the PID (personally identifiable data) Results Database 324b. These data are accessible for the authorized operators of the second server 320 via the Medical Officer Dashboard 321. In case of statistical data queries, the resulting data sets are subject to pre-processing, enabling the researcher to receive only statistical information gained from the query results.

These resulting data sets are then de-identified using the De-identification Engine 325 and also forwarded to the Data Storage module 324, more particularly to the Non-PID Results Database 324c. These de-identified data sets may be monitored by an authorized person through the IT Administrator Dashboard 322.

The Medical Officer, as an authorized operator of the second server 320, may release or deny the queried data via the Medical Officer Dashboard 321. At the same time, an IT Administrator of the second server 320 may monitor the de-identified data sets using the IT Administrator Dashboard 322.

Once released, the de-identified data sets are forwarded from the second server 320 through the Communication Gateway 327 to the first server 310, in which the de-identified data sets are received by the Communication Gateway 317 and then stored in the Data Storage 316, more particularly in the Query Results Storage 316b. The researcher can access the de-identified data sets through the Researcher Dashboard 310.

The query package formed by the Query Builder Module 314 may be a descriptor file that captures all information required to run the data query, namely the search criteria (inclusion /exclusion fields), custom mappings, the requested result configuration, and the scope of the requested query results / resources. The query packages may be represented in JSON format and edited using the query Builder Module 314. The various entities of the first server 310 allow to easily re-define the inclusion and exclusion criteria for the search. For example, the protocol requirements may be easily transformed into query search parameters with a technical descriptor. Such a descriptor is communicated through the system and only its status changes throughout the process.

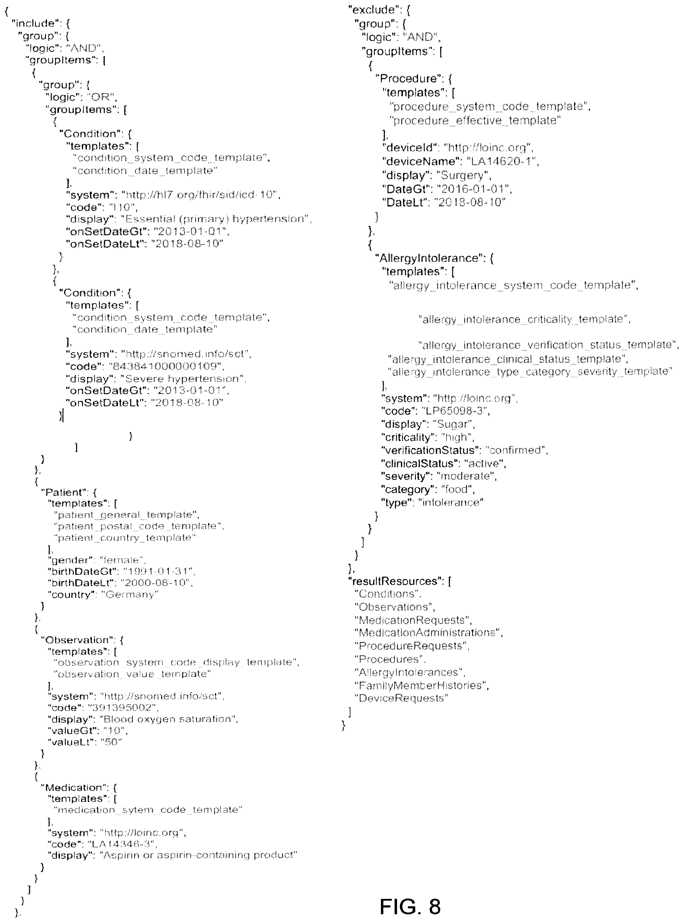

An example of the query descriptor is illustrated in Figure 8.

The Custom Mapping Handler 315 may be configured to allow the researcher to query clinical research sites' medical databases (Source Database 341) not only by using the nomenclature of the international medical code systems (e.g., ICD 10, SNOMED, LOINC, etc.), but also to search in free text format or via any local medical code bases as well. In this way the Custom Mapping Handler 315 preferably allows to establish logical correspondence between various medical code systems. In order to further broaden the scope of the query, free text tags / terms related to a specific disease (e.g. 'hypertension') may also be added as custom values.
These custom values may appear on the Researcher Dashboard 311 as follows:
'ICD10 / I10: *Essential (primary) hypertension* (custom value)
SNOMED / 843841000000109 : *Severe hypertension* (custom value)
BNO I10HO: '*Magasvernyomas*' (local medical code base, in Hungarian)
FREETEXT: *'hypertension'* (in English)
FREETEXT: *'hypertensio'* (in local vernacular)

Once the researcher has specified the complete value set for this custom array, the array is saved and forms a part of the researcher's custom code lists in the system, allowing the researcher to easily use it in future queries. When such a query is processed, the Custom Mapping Resolver 329 builds up elementary queries based on the incoming custom fields and values.

The aforementioned free text search capability allows the researcher to search for partial code strings or tags within a specific Source Database 341. By populating the FREETEXT custom value with a particular tag (e.g., 'lung cancer', etc.), the Query Builder Module 314 allows the researcher to locate all fields that contain the given expression in a given Source Database 341.

Research protocols may contain disease groups, not only individual ICD--10 or SNOMED codes, for example, as part of their inclusion / exclusion criteria. Some of the code systems are structured (e.g., ICD-10), but it is not easy to select a group of diseases with non-contiguous code values. The Custom Mapping Handler 315 may be configured to collect the individual disease codes into logically cohesive groups defined as custom fields / arrays. This approach can be applied to other resources as well, for example, in the case of either medications or substances.

Once the query 'fragments' have been processed and raw search results have been generated, the second server 320 starts assembling a final query result set that may be represented in a standard JSON format and then converted into a specific data structure as indicated below.
*SearchResult:*
   *FHIRSearchEngineAddress*
   *FHIRSearchEngine Version*
   *FHIRSearchEngineManualVersion*
      *Error*
   *TotalElement*
   *Identifier*
   *DetailedPatients (list):*
      *Patient(single resource)*
      *Conditions(list)*
         *Observations(list)*
      *MedicationAdministrations(list)*
      *Procedures(list)*
      *Allergylntolerances(list)*
         *FamilyMemberHistories(list)*
      *ProcedureRequests(list)*
      *DeviceRequests(list)*
      *MedicationRequests(list)*
      *Encounters(list)*
*SiteHeatmap*
   *SiteStatistics:*
      *Patients(number)*
      *Conditions(number)*
      *Observations(number)*
      *MedicationAdministrations(number)*
      *Procedures(number)*
      *Allergylntolerances(number)*
      *FamilyMemberHistories(number)*
      *ProcedureRequests(number)*
      *DeviceRequests(number)*
      *MedicationRequests(number)*
      *Encounters(number)*

In the above result structure, each item of the list represents a single resource. The researcher may define which resources are needed for his or her research purposes to help avoid overloading the servers and the communication channels unnecessarily. In this case, the nodes of the unselected resources should be left empty. Beyond the queried data elements, the query results may further contain administrative and generic information as well, including, for example, the server's version and IP address, potential error code, the number of returned elements (e.g. number of patients of interest), and the unique identifiers of the resulting data records. The above example illustrates a comprehensive query, meaning that the entire patient health graph is requested for research. Besides this, there may be other types of queries, like statistical queries and heatmap queries.

The statistical queries return the number of unique patients for a specific set of search criteria (along with the number of available resources at each resource grouping). The heatmap queries provide the researcher with a high level summary of the data available at a given Source Database 341, in particular the number of unique diagnoses, organized by the ICD-10 code, for example. In these special cases, the query results appear in the same structure, but the 'DetailedPatients' node is empty and, instead, the 'SiteHeatmap' or the 'SiteStatistics' node is populated.

The Conversion Module 328 and the Export Module 326 may be configured to transform the data query results into research-optimized formats to which the researchers are accustomed. The Conversion Module 328 and the Export Module 326 together serve as an output data connector of second server 320. The conversion and export functionality may support the JSON format, which is a standardized structured textual output format. The assembled result set may be converted to the researcher's proprietary data model or OMOP CDM v5.0.1 mapping, for example. The conversion algorithms can easily be adjusted to serve other data models as well.

The De-identification Engine 325 of the second server 320 may be configured to automatically and irreversibly de-identify personally identifiable data within (or as an extension to) the IT infrastructure of the second server 320, thus ensuring patient privacy at all times, as required by the respective legal regulations, for example the international standard ISO 25237:2017 on Health informatics (Pseudonymization).

In the above described embodiment of the recruitment system, the query IDs may be generated by the Query Manager 312 of the first server 310, the record IDs of the records returned in response to a given query may be generated by the Search Engine 331 of the second server 320. Alternatively, when the resulting data sets may be identified by unique anonymous IDs of the respective persons (e.g. patients), wherein the anonymous IDs are generated by the De-identification Engine 325.

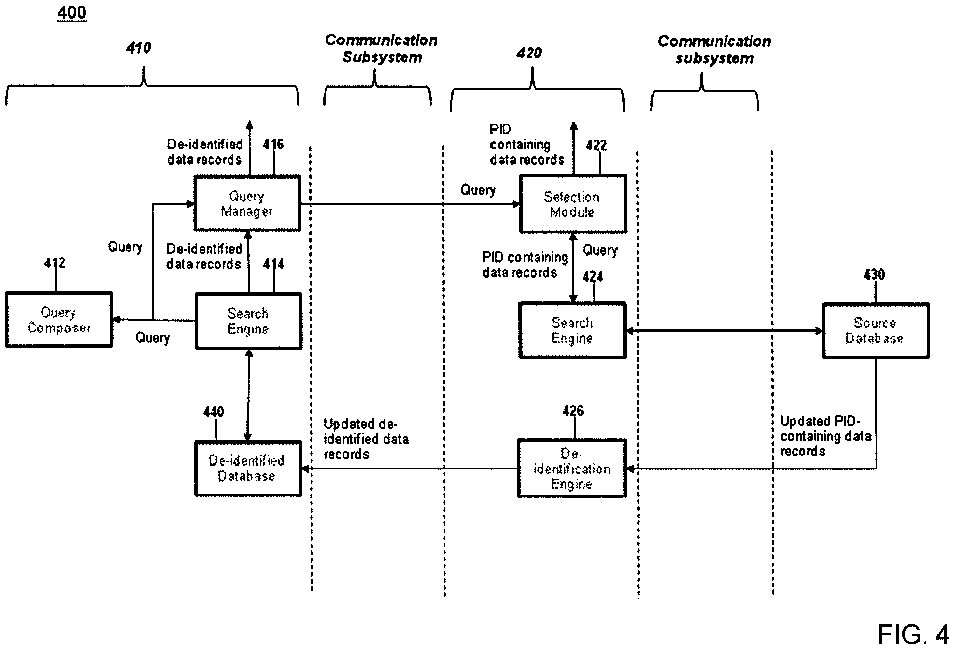

Figure 4 illustrates a schematic block diagram of a third embodiment of the recruitment system according to a second aspect of the present invention. In this recruitment system 400, a first server 420, a second server 410, a first database 430 containing a plurality of identifiable data records formed of application-specific person-related data and personally identifiable information, and a second database 440 containing a plurality of de-identified data records generated and updated on the basis of said first database 430.

The identifiable data records of the first database 430, serving as a source database, are stored without de-identification and only an authorized person of the first server 420 can access and use the content of the first database 430. This first database 430 may be an integral part of the first server 420, or may be coupled to the first server through a dedicated communication line or a communication subsystem, when the first database 430 is a local database residing at the site of the first server 420, or in some embodiments, the first database 430 may be connected to the first server 420 via a public communication network, like the internet, or a private communication network, like an intranet, when the first database 430 is not an integral part of the first server 420 or it is remote from the first server 420.

The de-identified data records of the second database 440 are periodically updated on the basis of the first database 430 and can be accessed only by an authorized user, e.g., a researcher, of the second server 410. The second database 440 is preferably arranged as an integral part of the second server 410, but it may also be remote from the second server 410. In this latter case, the second database 440 may be connected to the second server 410 via a communication subsystem, for example a public or private communication network.

Beyond the first database 440, the second server 410 further comprises at least a query composer 412, a search engine 414 and a query manager 416.

The query composer 412 is configured to provide a user interface for specifying queries for a search in the de-identified database 440 using the search engine 414. The query manager 416 is adapted for managing the queries, in particular for storing the executed queries and sending the current query to the first server 420 for the identification of the persons of interest. The query manager 416 is also adapted for receiving the de-identified data records resulted from a search performed by the search engine 414 based on a particular query. The resulted de-identified data records may be presented to an authorized person, e.g. a researcher, of the second server 410 for deciding whether the query is appropriate for sending it to the first server 420 for re-identifying one or more persons of interest.

When the second server 410 forwards an appropriate query to the first server 420, this query is received at the first server 420 by a selection module 422. The selection module is configured to obtain the identifiable data records from the source database, i.e. the first database 430, by means of a search engine 424 using the currently received query, and for presenting the identifiable data records for an authorized person of the first server 420, e.g., hospital physician, therapeutist, etc.

The first server 420 further comprises a de-identification engine 426 that is configured to monitor the first database 430 and in case of any modification of the first database 430, e.g., addition of a new data record, modification of any element of an existing data record, removal of a data record, etc., to update the content of the de-identified database 440 coupled to the second server 410. Updating includes the generation of a new de-identified data record on the basis of a new or modified data record of the first database 430, or removal of a de-identified data record from the second database 440 upon removal of the corresponding personally identifiable data record from the first (source) database 430.

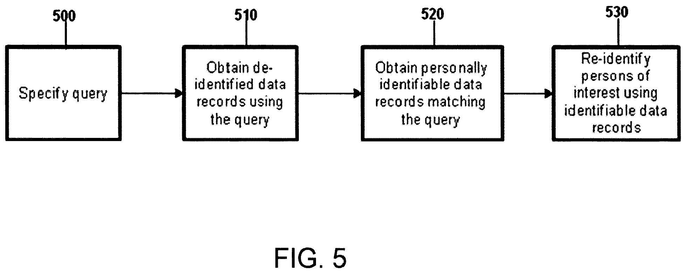

The method of identifying a person of interest using a database containing personally identifiable data according to the second aspect of the invention will now be described with reference to Figures 4 and 5, wherein Figure 5 illustrates a flow diagram including the essential steps of the method. Those terms used in relation with Figure 5, which are the same as used in relation with Figure 2, have the same meaning unless otherwise defined.

First, in step 500, a query for searching persons of interest in the de-identified database 440 is specified at the second server 410. The query may be specified using the query composer 412 and can be stored in the query manager 416 for future reference.

In the next step 510, de-identified data records are obtained from the de-identified database 440 based on the query. This step includes forwarding the query to the search engine 414 of the second server 410, which performs searching for de-identified data records in the de-identified database 440 based on the query, and sending the resulted one or more de-identified data records, if any, from the search engine 414 to the query manager 416.

If there is at least one de-identified data record matching the query, the corresponding query is forwarded to the first server 420 for obtaining the respective identifiable data records. If there is no matching data record in the de-identified database 440, the user of the second server 410 may be prompted to modify the query or to specify a new query.

Once the first server 420 has received a query from the second server 410, the at least one identifiable data record matching the query is retrieved from the source database 430 by means of the search engine 424 of the first server 420 in step 520. The resulted data records are then returned to the selection module 422, which may present the respective personally identifiable records to an authorized user of the first server 420.

Finally, in step 530, at least one person of interest is re-identified based on the at least one data record obtained from the first (source) database 430.

For the proper and reliable operation of the system and the method according to the second aspect of the present invention, it is necessary that the de-identified database 440 of the second server 410 always be consistent with the first source database 430 coupled to the first server 420 and containing personally identifiable data records. To this end, the de-identification engine 426 is responsible for maintaining the consistency between the two databases 430, 440.

In some embodiments of the method according to the second aspect of the present invention, the personally identifiable information of at least one person of interest may be obtained from the identifiable data record of the given person and may be communicated to the second server for further use by an end user of the second server who is authorized to use said personal data to contact the respective person for the purpose of his or her involvement in the recruitment program, like a clinical trial patient recruitment program.

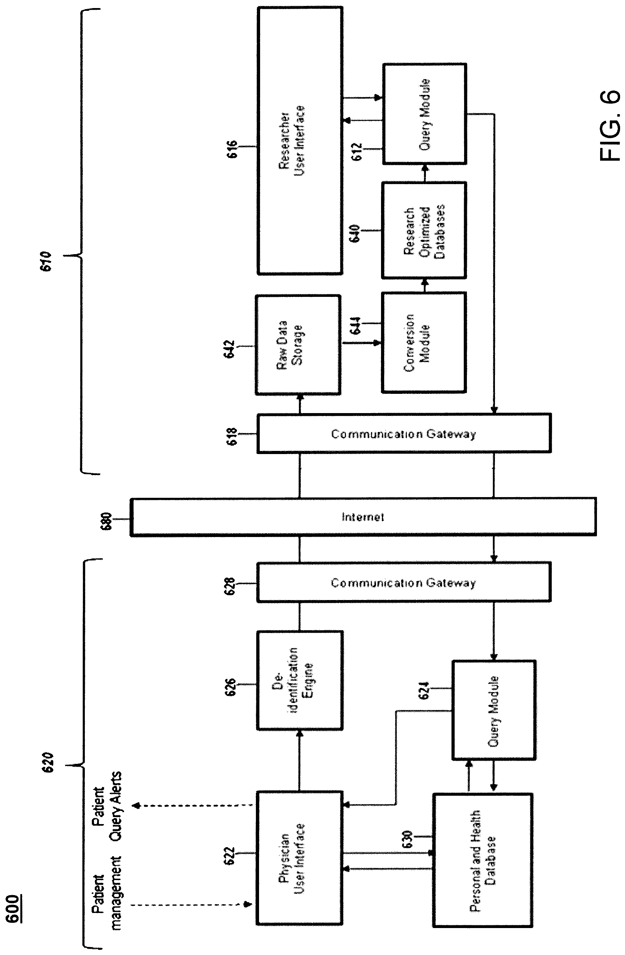

Figure 6 illustrates a fourth embodiment of the system according to the second aspect of the invention. This embodiment corresponds to a specific computer-implementation of the system in the medical field for a clinical trial recruitment scheme. As shown in Figure 6, the recruitment system 600 comprises a first server 620 and a second server 610 that are connected via the Internet 680. The secure communication between the first and second server 610, 620 over the Internet 680 is provided by the Communication Gateways 618 and 628, respectively.

The first server 620, which is operated, for example, by a physician comprises a Physician User Interface 622, a Query Module 624, a De-Identification Engine 626, a Communication Gateway 628 and a Personal & Health Database 630 serving as a source database for storing identifiable data records.

The second server 610, which is operated by an end-user, for example a researcher of a clinical research organization, comprises a Query Module 612, a researcher User Interface 616, a Research Optimized Database 640, a Raw Data Storage 642, a Conversion Module 642 and a Communication Gateway 618. In this embodiment of the system, the Research Optimized Database 640 functions as the de-identified database in which persons of interest are searched for by an authorized person of the second server 610 based on a particular query.

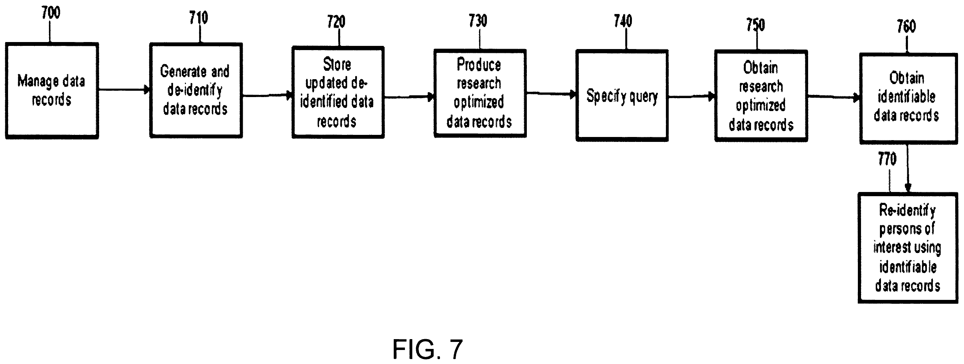

The operation of the system 600 shown in Figure 6 will be described below with reference to Figures 6 and 7, wherein Figure 7 is a flow diagram including the main steps of the method of identifying a person of interest using the system of Figure 6.

First, in step 700, the physician uses the first server 620 and its Personal & Health Database 630 to manage the data records of the database by means of the Physician User Interface 622. Managing the data records may include addition of new data records of new patients, modification of any one of the existing data records of patients having already registered in the database, or eventually removal of any one of the data records if needed.

When the physician performs an operation in the Personal & Health Database 630 with respect to a data record, either a new one or an existing one, the Anonymization Engine 626 automatically generates an updated data record and de-identifies the updated data record in step 710. The updated de-identified data record is then sent to the Raw Data Storage 642 of the second server 610 and stored therein in step 720. In this way the Raw Data Storage 642 is always maintained consistent with the source database, i.e. the Personal & Health Database 630 and it works as a de-identified mirrored database of the source database.

In step 730, the updated data records of the Raw Data Storage 642 are processed by the Conversion Module 644 to produce research-optimized data records for facilitating the search in the second server 610 operated by a researcher. The de-identified research-optimized data records are stored in the Research Optimized Database 640 serving as the de-identified database in which the researcher searches using a given query.

The queries are specified by the researcher using the Query Module 612 in step 740. By using the query, matching research-optimized data records are retrieved from the Research Optimized Database 640 in step 750. In case there is at least one matching data record that fits the needs of the researcher, the appropriate query will be sent to the first server 620 via the Internet 680.

In the first server 620, the query is received by the Query Module 624, which based on the query, obtains the matching identifiable data records from the Personal & Health Database 630 in step 760. The retrieved data records are then forwarded to the physician through the Physician User Interface 622 so that the physician can re-identify the person(s) of interest on the basis of the identifiable data record(s) thus obtained, and then contact the identified persons to inform them on the possibility of their involvement in the recruitment procedure of the clinical trials or other research.

The systems and the methods of the first aspect (a) and the second aspect (b) of the present invention are based on the following common inventive concept:
1. The end user (e.g. researcher) defines a search query.
2. A search is carried out
   a) in the source database; OR
   b) in the researcher's (de-identified) database
      using the search query.
3. The search result is
   a) received from the source database; OR
   b) produced locally
      in *anonymous* form.
4. The end user (e.g., researcher) sends record identification information to the source site in the form of
   a) Anonymous ID, or (query ID+record ID); OR
   b) the query itself.
5. The source site identifies the persons of interest by retrieving the identifiable data records of the selected persons from the source database
   a) using Anonymous ID, OR (query ID+record ID);
   b) running the query in the source database.

The only difference between the two aspects of the invention lies in the actual use of the databases by the end user, e.g. researcher, recruitment assistant, etc. In the first aspect of the invention, the search for the persons of interest is directly carried out once in the identifiable source database (first database), whereas in the second aspect, the search is first carried out in the de-identified database (second database) and then once more, in the source database (first database) for the re-identification of the persons of interest.

It is noted that within the context of the present invention, two or more entities of the system as described herein with reference to the drawings may be combined or integrated into one entity, or one entity of the system as described herein with reference to the drawings may be divided into two or more separate entities depending on the particular software and/or hardware implementation of the overall system of the present invention. All of these variants or modifications of the system fall into the scope of the invention as defined by the appended claims.

## Claims

**1.** A computer-implemented method of identifying a person of interest in a computerized recruitment system comprising a first server, a second server, a first database containing, for each person, a plurality of data records formed of application-specific person-related data and personally identifiable data associated with said person-related data, and a second database containing a plurality of de-identified data records, said second database being consistent with said first database, the method comprising:
a) specifying (200), at a first server, a query for searching persons of interest in the first database,
b) sending (210) the query to a search engine residing at a second server,
c) based on the query, obtaining (220), at the second server, at least one data record from the first database,
d) at the second server, storing (230) the personally identifiable data record results in the second database,
e) at the second server, de-identifying (240) the data records with generating a unique record identifier for each resulted data record,
f) returning (250) the de-identified data records with an associated unique record identifier to the first server,
g) selecting (260), at the first server, at least one data record from the de-identified data records according a to a predetermined selection scheme,
h) sending (270) the record identifier of the selected at least one de-identified data record to the second server,
i) at the second server, re-identifying (280) the selected at least one person of interest using the record identifier of the selected at least one de-identified data record and the set of personally identifiable data record results stored at the second server.

**2.** The method of claim 1, further comprising a step of communicating personally identifiable information of the at least one person of interest to the first server.

**3.** The method of claim 1 or 2, wherein the record identification information includes a query ID and a record ID belonging the said query ID.

**4.** The method of claim 1 or 2, wherein record identification information includes a unique anonymous ID (AID) of the person who is associated with the resulted data record, said anonymous ID being the same for each data record of a given person independently of the location of storage of the data record.

**5.** The method of claim 4, wherein generating the anonymous ID comprises the steps of:
- combining personally identifiable data elements of a data record into a single string,
- hashing the string with a salt value to produce a hashed value, and
- further encoding the hashed value to produce the anonymous ID.

**6.** The method of claim 5, further comprising storing (245) the set of de-identified data records with the associated record identifier at the second server.

**7.** The method of claim 6, wherein step e) of de-identifying the data records comprises anonymization or pseudonymization of the identifiable data records according to a predetermined scheme.

**8.** A computer-implemented method of identifying a person of interest in a computerized recruitment system comprising a first server, a second server, a first database containing, for each person, a plurality of data records formed of application-specific person-related data and personally identifiable data associated with said person-related data, and a second database containing a plurality of de-identified data records, said second database being consistent with said first database, the method comprising:
a) specifying (500), at the second server, a query for searching persons of interest in the second database,
b) based on the query, obtaining (510) de-identified data records from the second database,
c) at the first server, obtaining (520), from the first database, at least one personally identifiable data record matching the query, and
d) at the first server, re-identifying (530) at least one person of interest based on the at least one personally identifiable data record.

**8.** The method of claim 7, further comprising the step of communicating personally identifiable information of the at least one person of interest to the second server.

**9.** A computerized recruitment system (100) for identifying a person of interest, the system comprising:
a first server (110) configured to perform the steps a), b), g) and h) of the method of claim 1;
a second server (120) configured to perform the steps c) to f), and i) of the method of claim 1;
a first database (130) coupled to said first server (110) and containing, for each person, a plurality of data records formed of application-specific person-related data and personally identifying data associated with said person-related data, and
a second database (114) coupled to said second server (120) and containing a plurality of de-identified data records, said second database being consistent with said first database.

**10.** A computerized recruitment system (400) for identifying a person of interest, the system comprising:
a first server (420) configured to perform the steps c) and d) of the method of claim 8;
a second server (410) configured to perform the steps a) and b) of the method of claim 8;
a first database (430) coupled to said first server (420) and containing, for each person, a plurality of data records formed of application-specific person-related data and personally identifiable data associated with said person-related data, and
a second database (440) coupled to said second server (410) and containing a plurality of de-identified data records, said second database being consistent with said first database.
